# EUROPEAN PATENT SPECIFICATION

(11) **EP 0 643 965 B1**
(45) Date of publication and mention of the grant of the patent: **08.05.2013**
(21) Application number: 94306847.8
(22) Date of filing: 19.09.1994
(51) Int. Cl.: A61K 31/445, A61P 3/06

(54) **Nicotinic acid compositions for treating hyperlipidemia**
Nicotinsäure enthaltende Zusammensetzungen zur Behandlung der Hyperlipidämie
Compositions d'acide nicotinique pour le traitement de l'hyperlipidemie

(30) Priority: 20.09.1993 US 124392
(43) Date of publication of application: 22.03.1995
(60) Divisional application: 10075682.4
(73) Proprietor: Abbott Laboratories, Abbott Park, IL 60064 (US)
(72) Inventor: Bova, David J., Hollywood, Florida 33019 (US)
(74) Representative: Wallace, Sheila Jane

(56) References cited:
- EP-A- 0 349 235
- EP-A- 0 577 504
- US-A- 5 126 145
- Derwent Publications Ltd., London, GB; AN 89-036770 & JP-A-63 310 827 (SANWA KAGAKU)
- DATABASE WPI Derwent Publications Ltd., London, GB; AN 93-309117 & JP-A-5 221 854 (APSHER SMITH LAB.)
- J.CLIN.INVEST., vol.52, 1973 pages 732 - 40

## Description

This invention generally relates to compositions of nicotinic acid useful for treating hyperlipidemia. Methods of treating hyperlipidemia employing such compositions are also disclosed. More particularly, the present invention employs a composition of nicotinic acid, derivatives and mixtures thereof, and a swelling agent to form a time release sustaining composition for nocturnal or evening dosing. Specifically, the present invention employs a composition of nicotinic acid and hydroxypropyl methylcellulose to treat hyperlipidemia in a once per day oral dosage form given during the evening hours.

Nicotinic acid has been used for many years in the treatment of hyperlipidemia. This compound has long been known to exhibit the beneficial effects of reducing total cholesterol, low density lipoproteins or "LDL cholesterol", triglycerides and lipoprotein a (Lp(a)) in the human body, while increasing desirable high density lipoproteins or "HDL cholesterol".

Because of certain side effects however, nicotinic acid has normally been administered three times per day after meals. This dosing regimen is known to provide a very beneficial effect on blood lipids as discussed in Knopp et al; "Contrasting Effects of Unmodified and Time-Release Forms of Niacin on Lipoproteins in Hyperlipidemic Subjects: Clues to Mechanism of Action of Niacin"; Metabolism 34/7, 1985, page 647. The chief advantage of this profile is the ability of nicotinic acid to decrease total cholesterol, LDL cholesterol, triglycerides and Lp (a) while increasing HDL particles. While such a regimen does produce beneficial effects, cutaneous flushing and the like still often occurs in the hyperlipidemics to whom the compound is administered.

In order to avoid or reduce the undesirable side effects, a number of materials have been suggested for administration with an effective antihyperlipidemic amount of nicotinic acid, including guar gum in U.S. Pat. No. 4,965,252, and mineral salts as disclosed in U.S. Pat. No. 5,023,245; or inorganic magnesium salts as reported in U.S. Pat. No. 4,911,917. These materials have been shown to reduce various of the side effects commonly associated with nicotinic acid treatment.

Another method of avoiding or reducing the side effects associated with immediate release niacin is the use of sustained release formulations. Sustained release formulations are designed to slowly release the compound from the tablet or capsule. The slow drug release reduces and prolongs blood levels of drug and thus minimizes the side effects. Sustained release formulations of niacin have been developed, such as Nicobid™ capsules (Rhone-Poulenc Rorer), Endur-acin™ (Innovite Corporation) and Pat. No. 5,126,145 which describes a sustained release niacin formulation containing two different types of hydroxypropyl methylcellulose and a hydrophobic component.

Studies in hyperlipidemic patients have been conducted with a number of sustained release niacin products. These studies have demonstrated that the sustained release products do not have the same advantageous lipid altering effects as immediate release niacin, and in fact often have a worse side effect profile compared to the immediate release product. The major disadvantage of the sustained release formulations, as can be seen in Knopp et al., 1985, is the significantly lower reduction in triglycerides (-2% for the sustained release versus -38% for the immediate release) and lower increase in HDL cholesterol, represented as HDL₂ particles which are known by the art to be most beneficial, (-5% for the sustained release versus +37% for the immediate release).

Additionally, sustained release niacin formulations have been noted as causing greater incidences of liver toxicity as described in Henken et al (Am J Med 91:1991 1991) and Dalton et al (Am J Med 93: 102 1992). There is also great concern regarding the potential of these formulations in disrupting glucose metabolism and uric acid levels.

JP-A-6331 0827 discloses a slow release formulation of a nicotinic acid derivative containing a water soluble macromolecular substance, such as hydroxypropyl methyl cellulose that may be administered twice a day after a meal.

J. Clin. Invest, 52(3), 1973, 732-740 discloses that an intravenous infusion of niacin may be used to treat hyperlipidemia.

The Journal of Family Practice, 34, 1992, 313-319 discloses a wax-matrix controlled release formulation of nicotinic acid for use in combination with oat bran at doses of 1,500 mg and 56 g per day, respectively, wherein over 12% of the subjects suffered side effects or liver enzyme elevation following treatment with said combination.

Southern Medical Journal, 84, 1991, 496-497 discloses that ingestion of 3g per day of a long-acting nicotinic acid formulation caused lactic acidosis in a subject, resulting in the discontinuation of treatment.

Arch. Biochem. Biophys., 54, 1955, 558-559 discloses that administration of nicotinic acid four times a day may lower cholesterol levels, while causing facial flushing as a side effect.

JAMA, 261(24), 1989, 3582-3587 discloses the use of nicotinic acid to treat hyperlipidemia when administered in doses beginning at 2.5 to 3 g per day. It is noted that hepatitis and gastrointestinal distress are much more common side effects when nicotinic acid is administered as a sustained release formulation. However, side effects are minimised if the medication is always given with meals and if the initial dose is small (50mg three times a day) and is increased gradually over several weeks to the full therapeutic dose.

American Journal of Medicine, 92, 1992, 77-81 discloses that hepatic toxicity occurs more frequently with time-release preparations of niacin given two times a day than with unmodified preparations on a similar dosing schedule.

Therefore, it can be seen from the scientific literature that there is a need for development of a sustained release niacin formulation and a method of delivering said formulation which would provide hyperlipidemic patients with "balanced lipid alteration", i.e. reductions in total cholesterol, LDL cholesterol, triglycerides and Lp(a) as well as increases in HDL particles, with an acceptable safety profile, especially as regards liver toxicity and effects on glucose metabolism and uric acid levels.

It is known that in the human body, much of the body's cholesterol is synthesized during the evening and especially during periodic physiological loss of consciousness or "sleep". While the above discussed nicotinic acid compositions exhibit an ability to reduce total cholesterol, LDL cholesterol, triglycerides and Lp(a), as well as raising HDL cholesterol levels, they are all normally administered two-three times per day after meals. Hence, when the body's cholesterol and triglyceride synthesis mechanisms are at peak performance, nicotinic acid levels are at their lowest.

A need exists therefore, for a substained release formulation of nicotinic acid, and a method for the administration thereof, which can be administered once per day and which will substantially ensure that most of the nicotinic acid is released to the body during times of peak cholesterol synthesis while not producing any adverse effects on the liver, glucose metabolism or uric acid levels.

It is therefore, an object of the present invention to provide a composition of nicotinic acid or any compound which is metabolized by the body to form nicotinic acid for treating hyperlipidemia.

It is another object of the present invention to provide a composition as above, which has a time release sustaining characteristic.

A method for employing a composition as above, for treating hyperlipidemia, is also disclosed.

At least one or more of the foregoing objects, together with the advantages thereof over the known art relating to the treatment of hyperlipidemia, which shall become apparent from the specification which follows, are accomplished by the invention as hereinafter described and claimed.

According to one aspect of the present invention there is provided the use of nicotinic acid or a compound metabolized to nicotinic acid by the body selected from a group consisting of d-glucitol hexanicotinate, aluminium nicotinate, niceritrol, d,1-alpha-tocopheryl nicotinate and nicotinyl alcohol tartrate, for the manufacture of a sustained release medicament for use in the treatment by oral administration once per day prior to sleep, of hyperlipidaemia characterised in that the medicament does not comprise in admixture 5-30% hydroxypropyl methylcellulose, 2-15% of a water soluble pharmaceutical binder, 2-20% of a hydrophobic component and 30-90% nicotinic acid.

According to another aspect of the present invention there is provided a sustained release medicament comprising nicotinic acid or a compound metabolized to nicotinic acid by the body selected from a group consisting of d-glucitol hexanicotinate, aluminium nicotinate, niceritrol, d,1-alpha-tocopheryl nicotinate and nicotinyl alcohol tartrate, for use in the treatment by oral administration once per day prior to sleep, of hyperlipidaemia characterised in that the medicament does not comprise in admixture 5-30% hydroxypropyl methylcellulose, 2-15% of a water soluble pharmaceutical binder, 2-20% of a hydrophobic component and 30-90% nicotinic acid.

In general the present invention provides an improved antihyperlipidemia composition of the oral type employing an effective antihyperlipidemic amount of nicotinic acid, wherein the improvement comprises compounding the nicotinic acid with from about 5% to about 50% parts by weight of hydroxypropyl methylcellulose per hundred parts by weight of tablet or formulation.

The present invention also provides an orally administered antihyperlipidemia composition which comprises from about 30% to about 90% parts by weight of nicotinic acid; and, from about 5% to about 50% parts by weight of hydroxypropyl methylcellulose.

A method of treating hyperlipidemia in a hyperlipidemic having a substantially periodic physiological loss of consciousness is also disclosed. The method comprises the steps of forming a composition which comprises an effective antihyperlipidemic amount of nicotinic acid and an amount of excipients to provide sustained release of drug. The method also includes the step of orally administering the composition to the hyperlipidemic prior to each periodic physiological loss of consciousness.

The present invention employs nicotinic acid or a compound other than nicotinic acid itself which the body metabolizes into nicotinic acid, thus producing the same effect as described herein. The other compounds specifically include the following: nicotinyl alcohol tartrate, d-glucitol hexanicotinate, aluminum nicotinate, niceritrol and d,1-alpha-tocopheryl nicotinate. Each such compound will be collectively referred to hereinbelow by "nicotinic acid."

As stated hereinabove, nicotinic acid has been employed in the past for the treatment of hyperlipidemia, which condition is characterized by the presence of excess fats such as cholesterols and triglycerides, in the blood stream. According to the present invention, a sustained release composition of nicotinic acid is prepared as an example. By "sustained release" it is understood to mean a composition which when orally administered to a patient to be treated, the active ingredient will be released for absorption into the blood stream over a period of time. For example, it is preferred that in a dosage of about 1500 milligrams (hereinafter "mgs") of nicotinic acid, approximately 100 percent of the nicotinic acid will be released to the blood stream in about 6 to about 24 hours.

The specific sustained release composition according to the present invention employs an effective antihyperlipidemic amount of nicotinic acid. By "effective antihyperlipidemic amount" it is understood to mean an amount which when orally administered to a patient to be treated, will have a beneficial effect upon the physiology of the patient, to include at least some lowering of total cholesterol, LDL cholesterol, triglycerides and Lp(a) and at least some increase in HDL cholesterol in the patient's blood stream. An exemplary effective antihyperlipidemic amount of nicotinic acid would be from about 250 mgs to about 3000 mgs of nicotinic acid to be administered according to the invention as will be more fully described hereinbelow. This amount will vary dependent upon a number of variables, including the physiological needs of the patient to be treated.

Preferably, there is also included in the sustained release composition according to the present invention, a swelling agent which is compounded with the nicotinic acid, such that when the composition is orally administered to the patient, the swelling agent will swell over time in the patient's gastrointestinal tract, and release the active nicotinic acid, or a compound which produces nicotinic acid into the gastrointestinal system for absorption into the blood stream, over a period of time. As is known in the art, such swelling agents and amounts thereof, may be preselected in order to control the time release of the active ingredient. Such swelling agents include polymers such as sodium carboxymethylcellulose and ethylcellulose and waxes such as bees wax and natural materials such as gums and gelatins or mixtures of any of the above. Because the amount of the swelling agent will vary depending upon the nature of the agent, the time release needs of the patient, it is preferred to employ amounts of the agent which will accomplish the objects of the invention.

An exemplary and preferred swelling agent is hydroxypropyl methylcellulose, in an amount ranging from about 5% to about 50% parts by weight per 100 parts by weight of tablet or formulation. The preferred example will ensure a sustained time release over a period of approximately 6-24 hours as demonstrated by in vitro dissolution techniques known to the art.

A binder may also be employed in the present compositions. While any known binding material is useful in the present invention, it is preferred to employ a material such as one or more of a group of polymers having the repeating unit of 1-ethenyl-2-pyrrolidinone. These polymers generally have molecular weights of between about 10,000 and 700,000, and are also known as "povidone".

Amounts of the binder material will of course, vary depending upon the nature of the binder and the amount of other ingredients of the composition. An exemplary amount of povidone in the present compositions would be from about 1% to about 5% by weight of povidone per 100 parts by weight of the total formulation.

Processing aids such as lubricants, including stearic acid, may also be employed, as is known in the art. An exemplary amount of stearic acid in the present compositions would be from about 0.5% to about 2.0% by weight per 100 parts by weight of tablet or formulation.

As was discussed hereinabove, it is known that much of the lipids in the human body are produced therein during the evening hours and especially during episodes of periodic physiological loss of consciousness or "sleep". By "periodic" it is understood to mean in a substantially uniform repeating pattern. For example, an adult may sleep approximately 8 hours per day. By being administered prior to each periodic physiological loss of consciousness, that is, orally administering the composition during the evening after dinner or as the person lies down to go to sleep, the present composition is capable of providing effective antihyperlipidemic amounts of nicotinic acid to the body during times of peak lipid production or synthesis.

Furthermore, because the composition is orally administered prior to sleep, larger amounts of the active ingredient nicotinic acid is available during lipid synthesis times than if the composition is administered during hours when the patient is awake, i.e., in the morning or afternoon hours. Hence, less nicotinic acid may be required to be administered than would otherwise be required with compositions heretofore known in the art, while achieving substantially similar or even superior antihyperlipidemia results for at least one or more of the lipoproteins or particles discussed above. Because less nicotinic acid is administered and because the invention specifically describes once a day dosing, there is a lesser amount of the side effects as discussed hereinabove. The time release sustaining compositions according to the present invention ensure that effective antihyperlipidemia amounts of nicotinic acid are released to the patient during times of lipid production.

### General Experimental

In order to demonstrate the effectiveness of the compositions and method of the present invention over known antihyperlipidemia compositions and methods heretofore known in the art, a number of substantially identical composition were prepared according to the disclosure hereinabove. The composition ingredients and amounts are listed in TABLE I hereinbelow.

**TABLE I**

| **Test Tablet Composition** | | | |
|---|---|---|---|
| Ingredient | 375 mg | 500 mg | 750 mg |
| Nicotinic Acid | 375.0 | 500.0 | 750.0 |
| Hyroxypropyl methylcellulose | 188.7 | 203.0 | 204.7 |
| Povidone | 12.9 | 17.2 | 25.9 |
| Stearic Acid | 5.8 | 7.3 | 9.9 |
| | | | |
| **TOTAL** | 582.4 mg | 727.5 mg | 990.5 mg |

The ingredients were compounded together to form a tablet. Two study groups consisting of eleven and fourteen patients each were formed. Blood samples were taken from the patients, and tested for total cholesterol, LDL cholesterol, triglycerides and HDL cholesterol to establish baseline levels from which fluctuations in these lipids could be compared. The patients were then placed upon a regimen of the above discussed tablets, totaling approximately 1500 mg of nicotinic acid, once per day before going to bed. After eight weeks of this regimen, the patients were again tested for lipid profiles. The results of the tests conducted at eight weeks, showing the changes in the lipid profiles as a percentage change from the baseline, are reported in the table hereinbelow. Positive numbers reflect percentage increases and negative numbers reflect percentage decreases in this table.

**TABLE II**

| **Patient Study Lipid Profile Data** | | | | | | | |
|---|---|---|---|---|---|---|---|
| **GROUP A** | | | | | | | |
| Pt. No. | Total-C | LDL-C | Apo B | Trigs | HDL-C | HDL₂-C | Lp(a) |
| 1 | -8.2 | -12.0 | NA | -17.3 | 22.0 | NA | NA |
| 2 | -5.9 | -27.0 | NA | -28.7 | 65.0 | NA | NA |
| 3 | -15.1 | -13.0 | NA | -22.0 | -9.1 | NA | NA |
| 4 | -3.3 | -10.0 | NA | 61.6 | 3.8 | NA | NA |
| 5 | -16.5 | -17.7 | NA | -28.8 | 11.1 | NA | NA |
| 6 | -12.4 | -25.9 | NA | -42.0 | 51.6 | NA | NA |
| 7 | -24.2 | -31.4 | NA | -39.4 | 12.5 | NA | NA |
| 8 | -6.7 | -7.4 | NA | -42.4 | 18.8 | NA | NA |
| 9 | 4.5 | 1.1 | NA | 7.2 | 9.2 | NA | NA |
| 10 | 2.8 | -0.2 | NA | -2.7 | 22.9 | NA | NA |
| 11 | -13.0 | -9.4 | NA | -54.0 | 44.3 | NA | NA |
| | | | | | | | |
| Mean | -8.9 | -13.9 | NA | -18.9 | 23.0 | NA | NA |
| p-Value | 0.0004 | 0.0001 | | 0.0371 | 0.0068 | | |
| | | | | | | | |

| **GROUP B** | | | | | | | |
|---|---|---|---|---|---|---|---|
| 1 | -19.2 | -27.1 | -24.4 | -33.4 | 20.0 | 22.3 | -81.9 |
| 2 | -32.2 | -35.7 | -28.0 | -60.4 | 4.3 | 3.2 | -25.3 |
| 3 | -21.4 | -33.6 | -35.6 | -33.4 | 30.4 | 38.6 | -17.4 |
| 4 | -19.9 | -24.6 | -15.1 | -20.8 | 9.6 | 16.1 | -27.0 |
| 5 | -3.3 | -2.1 | -29.4 | -41.1 | 5.8 | 2.4 | -22.4 |
| 6 | PATIENT WITHDREW FROM STUDY | | | | | | |
| 7 | 23.1 | -32.6 | -42.6 | -58.6 | 49.2 | 68.9 | -14.3 |
| 8 | 24.8 | 34.0 | -28.4 | 5.5 | 6.5 | -6.8 | NA |
| 9 | 10.1 | 12.0 | -16.8 | -11.6 | 20.7 | -12.3 | 40.6 |
| 10 | -2.9 | -7.7 | -28.0 | -59.0 | 53.1 | 70.5 | -41.2 |
| 11 | -10.5 | -18.8 | -25.3 | -53.4 | 31.8 | 39.7 | NA |
| 12 | -20.0 | -30.8 | -30.4 | 11.7 | 21.1 | 25.0 | -28.4 |
| 13 | 17.4 | 16.8 | -17.5 | -17.5 | 51.3 | 51.9 | 38.5 |
| 14 | -9.4 | -16.6 | -32.0 | -46.9 | 52.3 | 67.6 | 17.6 |
| Mean | -8.7 | -12.8 | -32.2 | -27.2 | 25.3 | 30.1 | -17.9 |
| p-Value | 0.0002 | <0.0001 | 0.0001 | <0.001 | <0.0001 | 0.0002 | <0.0188 |
| | | | | | | | |
| Combined | -8.7 | -13.3 | Gp B | -26.1 | 25.3 | Gp B | Gp B |
| p-Value | 0.0002 | <0.0001 | only | <.0001 | <0.0001 | only | only |

The data reported in TABLE II shows that the LDL levels in the Group A patients had a mean decrease of -13.9% and triglyceride decrease of -18.9%. HDL cholesterol levels, the beneficial cholesterol, were raised by 23.0% in this Group. Similar results were obtained with the Group B patients. These studies demonstrate that dosing the sustained release formulation during the evening hours or at night provides reductions in LDL cholesterol levels equal to immediate release niacin on a milligram per milligram basis, but superior reductions in triglyceride reductions when compared to sustained release formulations dosed during daytime hours on a milligram per milligram basis. Additionally, the increases in HDL cholesterol obtained from dosing the sustained release formulation during the evening or at night were +23.0% for one group and +25.3% for the other group. Dosing during the evening therefore provides reduction in LDL cholesterol plus significant decreases in triglycerides and increases in HDL cholesterol with once-a-day dosing.

Groups A and B were also tested for liver enzymes (AST, ALT and Alkaline Phosphatase), uric acid and fasting glucose levels at the start of the study described hereinabove (to form a baseline) and at two, four and eight week intervals. The results of these tests are listed in TABLES III-VII hereinbelow.

**TABLE III**

| **THE EFFECT OF NIASPAN^{™} THERAPY ON AST (SGOT) LEVELS (U/L)** (1500 mgs dosed once-a-day at night) (n = 28) | | | | | |
|---|---|---|---|---|---|
| | | | | | |

| | Weeks Of Therapy With NIASPAN^{™} | | | | |
|---|---|---|---|---|---|
| Pt # | Baseline | 2 Wks. | 4 Wks. | 8 Wks. | Reference Range |
| **GROUP A** | | | | | |
| 1 | 28 | 29 | 25 | 24 | 0-50 |
| 2 | 24 | 25 | 24 | 26 | 0-50 |
| 3 | 17 | 18 | 22 | 21 | 0-50 |
| 4 | 14 | 16 | 15 | 17 | 0-50 |
| 5 | 22 | NA | 32 | 52 | 0-50 |
| 6 | 21 | 17 | 17 | 14 | 0-50 |
| 7 | 17 | 17 | 14 | 18 | 0-50 |
| 8 | 20 | 21 | 22 | 22 | 0-50 |
| 9 | 16 | 16 | 17 | 20 | 0-50 |
| 10 | 18 | 21 | 21 | 25 | 0-50 |
| 11 | 21 | 21 | 22 | 21 | 0-50 |
| | | | | | |

| **GROUP B** | | | | | |
|---|---|---|---|---|---|
| 1 | 23 | 25 | 38 | 33 | 0-50 |
| 2 | 20 | 20 | 21 | 21 | 0-50 |
| 3 | 15 | 20 | 18 | 19 | 0-50 |
| 4 | 25 | 22 | 25 | 26 | 0-50 |
| 5 | 23 | 21 | 17 | 18 | 0-50 |
| 6 | PATIENT WITHDREW DUE TO FLUSHING | | | | |
| 7 | 21 | 18 | 18 | 19 | 0-50 |
| 8 | 18 | 19 | 18 | 19 | 0-50 |
| 9 | 15 | 16 | 18 | 15 | 0-50 |
| 10 | 16 | 15 | 19 | 28 | 0-50 |
| 11 | 20 | 22 | 24 | 28 | 0-50 |
| 12 | 23 | 25 | 28 | 22 | 0-50 |
| 13 | 20 | 15 | 20 | 19 | 0-50 |
| 14 | 18 | 25 | 20 | 18 | 0-50 |
| | | | | | |
| Combined Mean | 19.8 | 20.4 | 20.8 | 21.1 | |
| | | | | | |
| Change From Baseline | | +3.0% | +5.1% | +6.6% | |

| | | | | | |
|---|---|---|---|---|---|
| Level of Significance: p=0.4141 | | | | | |

**TABLE IV**

| **THE EFFECT OF NIASPAN^{™} THERAPY ON ALT (SGPT) LEVELS (U/L)** (1500 mgs dosed once-a-day at night) (n = 28) | | | | | |
|---|---|---|---|---|---|
| | | | | | |

| | Weeks Of Therapy With NIASPAN^{TM} | | | | |
|---|---|---|---|---|---|
| Pt # | Baseline | 2 Wks. | 4 Wks. | 8 Wks. | Reference Range |
| **GROUP A** | | | | | |
| 1 | 32 | 28 | 39 | 30 | 0-55 |
| 2 | 24 | 25 | 23 | 26 | 0-55 |
| 3 | 18 | 23 | 30 | 30 | 0-55 |
| 4 | 7 | 13 | 14 | 14 | 0-55 |
| 5 | 14 | NA | 43 | 46 | 0-55 |
| 6 | 22 | 11 | 14 | 10 | 0-55 |
| 7 | 9 | 7 | 11 | 7 | 0-55 |
| 8 | 16 | 18 | 23 | 21 | 0-55 |
| 9 | 14 | 17 | 20 | 14 | 0-55 |
| 10 | 14 | 15 | 17 | 19 | 0-55 |
| 11 | 18 | 18 | 20 | 16 | 0-55 |
| | | | | | |

| **GROUP B** | | | | | |
|---|---|---|---|---|---|
| 1 | 16 | 17 | 27 | 29 | 0-55 |
| 2 | 16 | 14 | 15 | 22 | 0-55 |
| 3 | 13 | 21 | 13 | 16 | 0-55 |
| 4 | 23 | 20 | 26 | 17 | 0-55 |
| 5 | 21 | 23 | 17 | 15 | 0-55 |
| 6 | PATIENT WITHDREW DUE TO FLUSHING | | | | |
| 7 | 21 | 16 | 18 | 21 | 0-55 |
| 8 | 18 | 20 | 17 | 18 | 0-55 |
| 9 | 11 | 5 | 11 | 8 | 0-55 |
| 10 | 8 | 10 | 14 | 17 | 0-55 |
| 11 | 17 | 12 | 18 | 16 | 0-55 |
| 12 | 14 | 18 | 20 | 16 | 0-55 |
| 13 | 14 | NA | 11 | 10 | 0-55 |
| 14 | 23 | 23 | 19 | 19 | 0-55 |
| | | | | | |
| Combined Mean | 17.7 | 17.5 | 19.3 | 18.2 | |
| Change From Baseline | | -1.1% | 9.0% | +2.8% | |

| | | | | | |
|---|---|---|---|---|---|
| Level of Significance: p=0.3424 | | | | | |

**TABLE V**

| **THE EFFECT OF NIASPAN^{™} THERAPY ON ALKALINE PHOSPHATASE LEVELS (U/L)** (1500 mgs dosed once-a-day at night) (n = 28) | | | | | |
|---|---|---|---|---|---|
| | | | | | |

| | Weeks Of Therapy With NIASPAN^{™} | | | | |
|---|---|---|---|---|---|
| Pt # | Baseline | 2 Wks. | 4 Wks. | 8 Wks. | Reference Range |
| **GROUP A** | | | | | |
| 1 | 52 | 56 | 57 | 55 | 20-140 |
| 2 | 103 | 100 | 89 | 102 | 20-140 |
| 3 | 54 | 45 | 53 | 51 | 20-140 |
| 4 | 70 | 68 | 71 | 91 | 20-140 |
| 5 | 77 | NA | 74 | 81 | 20-140 |
| 6 | 55 | 48 | 49 | 51 | 20-140 |
| 7 | 72 | 71 | 79 | 75 | 20-140 |
| 8 | 55 | 49 | 47 | 50 | 20-140 |
| 9 | 53 | 55 | 56 | 45 | 20-140 |
| 10 | 74 | 73 | 75 | 75 | 20-140 |
| 11 | 18 | 18 | 20 | 16 | 20-140 |
| | | | | | |

| **GROUP B** | | | | | |
|---|---|---|---|---|---|
| 1 | 73 | 67 | 89 | 95 | 20-140 |
| 2 | 82 | 64 | 72 | 71 | 20-140 |
| 3 | 73 | 69 | 72 | 82 | 20-140 |
| 4 | 37 | 36 | 37 | 38 | 20-140 |
| 5 | 65 | 53 | 54 | 61 | 20-140 |
| 6 | PATIENT WITHDREW DUE TO FLUSHING | | | | |
| 7 | 64 | 58 | 58 | 58 | 20-140 |
| 8 | 79 | 78 | 65 | 73 | 20-140 |
| 9 | 94 | 92 | 103 | 93 | 20-140 |
| 10 | 69 | 67 | 70 | 65 | 20-140 |
| 11 | 59 | 67 | 63 | 72 | 20-140 |
| 12 | 65 | 59 | 59 | 63 | 20-140 |
| 13 | 64 | 68 | 66 | 64 | 20-140 |
| 14 | 72 | 61 | 59 | 64 | 20-140 |
| | | | | | |
| Combined Mean | 65.5 | 61.5 | 63.3 | 65.8 | |
| Change From Baseline | | -6.1% | -3.4% | +0.005% | |

| | | | | | |
|---|---|---|---|---|---|
| Level of Significance: p=0.0236 | | | | | |

**TABLE VI**

| **THE EFFECT OF NIASPAN^{™} THERAPY ON URIC ACID LEVELS (mg/dL)** (1500 mgs dosed once-a-day at night) (n = 28) | | | | | |
|---|---|---|---|---|---|
| | | | | | |

| | Weeks Of Therapy With NIASPAN^{™} | | | | |
|---|---|---|---|---|---|
| Pt # | Baseline | 2 Wks. | 4 Wks. | 8 Wks. | Reference Range |
| **GROUP A** | | | | | |
| 1 | 5.2 | 5.0 | 4.8 | 4.3 | 4.0-8.5 |
| 2 | 4.0 | 4.6 | 4.5 | 6.2 | 2.5-7.5 |
| 3 | 6.3 | 7.0 | 6.5 | 6.2 | 4.0-8.5 |
| 4 | 3.1 | 4.6 | 4.2 | 3.8 | 2.5-7.5 |
| 5 | 3.4 | NA | 3.3 | 4.2 | 2.5-7.5 |
| 6 | 6.6 | 5.5 | 5.6 | 4.7 | 4.0-8.5 |
| 7 | 3.8 | 4.5 | 4.3 | 4.9 | 2.5-7.5 |
| 8 | 4.4 | 3.8 | 5.1 | 4.5 | 2.5-7.5 |
| 9 | 3.9 | 4.5 | 4.6 | 3.5 | 2.5-7.5 |
| 10 | 2.6 | 2.9 | 2.8 | 2.7 | 2.5-7.5 |
| 11 | 4.7 | 5.5 | 5.2 | 5.3 | 2.5-7.5 |
| | | | | | |

| **GROUP B** | | | | | |
|---|---|---|---|---|---|
| 1 | 3.7 | 4.2 | 4.7 | 3.5 | 2.5-7.5 |
| 2 | 2.8 | 3.5 | 3.6 | 2.3 | 4.0-8.5 |
| 3 | 4.2 | 5.3 | 5.5 | 5.3 | 2.5-7.5 |
| 4 | 4.7 | 3.9 | 5.1 | 3.6 | 4.0-8.5 |
| 5 | 3.7 | 4.1 | 4.1 | 3.8 | 2.5-7.5 |
| 6 | PATIENT WITHDREW DUE TO FLUSHING | | | | |
| 7 | 5.8 | 6.6 | 6.6 | 6.8 | 2.5-7.5 |
| 8 | 4.7 | 4.3 | 5.4 | 5.6 | 2.5-7.5 |
| 9 | 3.7 | 4.6 | 5.1 | 3.8 | 2.5-7.5 |
| 10 | 4.2 | 5.0 | 4.4 | 8.5 | 2.5-7.5 |
| 11 | 1.9 | 3.0 | 2.8 | 5.0 | 2.5-7.5 |
| 12 | 5.6 | 5.4 | 6.2 | 5.6 | 4.0-8.5 |
| 13 | 4.2 | 4.6 | 4.6 | 5.3 | 2.5-7.5 |
| 14 | 5.5 | 5.4 | 6.1 | 5.3 | 2.5-7.5 |
| | | | | | |
| Combined Mean | 4.54 | 4.82 | 4.92 | 4.86 | *p=0.3450 |
| Change From Baseline | | +6.2% | +8.4% | +7.0% | |

| | | | | | |
|---|---|---|---|---|---|
| *Level of Significance: p=0.3450 | | | | | |

**TABLE VII**

| **THE EFFECT OF NIASPAN^{™} THERAPY ON FASTING GLUCOSE LEVELS (mg/dL)** (1500 mgs dosed once-a-day at night) (n = 28) | | | | | |
|---|---|---|---|---|---|
| | | | | | |

| | Weeks Of Therapy With NIASPAN^{™} | | | | |
|---|---|---|---|---|---|
| Pt # | Baseline | 2 Wks. | 4 Wks. | 8 Wks. | Reference Range |
| **GROUP A** | | | | | |
| 1 | 114 | 122 | 123 | 110 | 70-115 |
| 2 | 101 | 105 | 107 | 101 | 80-125 |
| 3 | 99 | 98 | 109 | 103 | 70-115 |
| 4 | 100 | 118 | 94 | 94 | 80-125 |
| 5 | 89 | NA | 82 | 103 | 80-125 |
| 6 | 97 | 103 | 94 | 107 | 70-115 |
| 7 | 85 | 107 | 100 | 94 | 80-125 |
| 8 | 98 | 107 | 103 | 101 | 80-125 |
| 9 | 97 | 97 | 100 | 110 | 80-125 |
| 10 | 94 | 101 | 111 | 97 | 70-115 |
| 11 | 102 | 103 | 95 | 95 | 80-125 |
| | | | | | |

| **GROUP B** | | | | | |
|---|---|---|---|---|---|
| 1 | 101 | 97 | 83 | 99 | 70-115 |
| 2 | 90 | 95 | 96 | 89 | 80-125 |
| 3 | 96 | 98 | 95 | 97 | 70-115 |
| 4 | 116 | 139 | 113 | 125 | 80-125 |
| 5 | 88 | 92 | 91 | 95 | 70-115 |
| 6 | PATIENT WITHDREW DUE TO FLUSHING | | | | |
| 7 | 106 | 114 | 118 | 117 | 70-115 |
| 8 | 95 | 106 | 106 | 108 | 70-115 |
| 9 | 81 | 92 | 84 | 92 | 70-115 |
| 10 | 108 | 117 | 122 | 105 | 70-115 |
| 11 | 85 | 106 | 106 | 108 | 70-115 |
| 12 | 92 | 89 | 101 | 86 | 80-125 |
| 13 | 99 | 105 | 94 | 100 | 70-125 |
| 14 | 100 | 108 | 84 | 107 | 70-125 |
| | | | | | |
| Combined Mean | 98.4 | 105.8 | 101.6 | 102.3 | |
| Change From Baseline | | +7.5% | +3.3% | +4.0% | |

| | | | | | |
|---|---|---|---|---|---|
| Level of Significance: p=0.0021 | | | | | |

These results indicate that this sustained release dosage form caused no elevation in liver function tests (i.e., no liver damage), no elevations in uric acid and only a small, 7.5% increase in fasting glucose levels which in fact decreased during continued therapy.

Thus it should be evident that the compositions of the present invention and the method disclosed herein are highly effective in controlling hyperlipidemia in hyperlipidemics, by reducing the levels of LDL cholesterol, triglyceride and Lp(a) while increasing HDL cholesterol levels. The present invention is also demonstrated not to cause elevations in liver function tests, uric acid or glucose levels for the hyperlipidemics.

Based upon the foregoing disclosure, it should now be apparent that the use of the compositions and methods described herein will carry out the objects set forth hereinabove.

## Claims

1. The use of nicotinic acid or a compound metabolized to nicotinic acid by the body selected from a group consisting of d-glucitol hexanicotinate, aluminium nicotinate, niceritrol, d,1-alpha-tocopheryl nicotinate and nicotinyl alcohol tartrate, for the manufacture of a sustained release medicament for use in the treatment by oral administration once per day prior to sleep, of hyperlipidaemia **characterised in that** the medicament does not comprise in admixture 5-30% hydroxypropyl methylcellulose, 2-15% of a water soluble pharmaceutical binder, 2-20% of a hydrophobic component and 30-90% nicotinic acid.

2. Use according to claim 1, wherein said sustained release medicament comprises from 250 milligrams to 3000 milligrams of nicotinic acid or a compound metabolised to nicotinic acid by the body selected from a group consisting of d-glucitol hexanicotinate, aluminium nicotinate, niceritrol, d,1-alpha-tocopheryl nicotinate and nicotinyl alcohol tartrate.

3. Use according to any one of the preceding claims, wherein said sustained release medicament comprises from 5% to 50% parts by weight of hydroxypropyl methylcellulose.

4. Use according to any one of the preceding claims, wherein said sustained release medicament comprises from 1% to 4% by weight of a pharmaceutically acceptable binder.

5. Use according to claim 4, wherein said binder is a polymer having the repeating unit 1-ethenyl-2-pyrrolidone.

6. Use according to any one of the preceding claims, wherein said sustained release medicament comprises from 0.5 to 2.0 parts by weight of a lubricant.

7. Use according to claim 6, **characterised in that** said lubricant is stearic acid or magnesium stearate.

8. A sustained release medicament comprising nicotinic acid or a compound metabolized to nicotinic acid by the body selected from a group consisting of d-glucitol hexanicotinate, aluminium nicotinate, niceritrol, d,1-alpha-tocopheryl nicotinate and nicotinyl alcohol tartrate, for use in the treatment by oral administration once per day prior to sleep, of hyperlipidaemia **characterised in that** the medicament does not comprise in admixture 5-30% hydroxypropyl methylcellulose, 2-15% of a water soluble pharmaceutical binder, 2-20% of a hydrophobic component and 30-90% nicotinic acid.

9. A sustained release medicament according to claim 8, wherein said sustained release medicament comprises from 250 milligrams to 3000 milligrams of nicotinic acid or a compound metabolised to nicotinic acid by the body selected from a group consisting of d-glucitol hexanicotinate, aluminium nicotinate, niceritrol, d,1-alpha-tocopheryl nicotinate and nicotinyl alcohol tartrate.

10. A sustained release medicament according to claim 8 or claim 9, wherein said sustained release medicament comprises from 5% to 50% parts by weight of hydroxypropyl methylcellulose.

11. A sustained release medicament according to any one of claims 8 to 10, wherein said sustained release medicament comprises from 1% to 4% by weight of a pharmaceutically acceptable binder.

12. A sustained release medicament according to claim 11, wherein said binder is a polymer having the repeating unit 1-ethenyl-2-pyrrolidone.

13. A sustained release medicament according to any one of claims 8 to 12, wherein said sustained release medicament comprises from 0.5 to 2.0 parts by weight of a lubricant.

14. A sustained release medicament according to claim 13, **characterised in that** said lubricant is stearic acid or magnesium stearate.

## Patentansprüche

1. Einsatz von Nicotinsäure oder einer aus der Gruppe von d-Glucitolhexanicotinat, Aluminiumnicotinat, Niceritrol, d,1-Alphatocopherylnicotinat und Nicotinylalkoholtartrat gewählten, durch den Körper zu Nicotinsäure verstoffwechselten Verbindung zur Herstellung eines Medikaments mit verzögerter Freisetzung zum Einsatz bei der Behandlung im Wege der oralen Verabreichung einmal pro Tag vor dem Schlafen von Hyperlipidämie, dadurch charakterisiert, dass das Medikament keine Beimischung von 5 - 30 % Hydroxypropylmethylcellulose, 2-15 % eines wasserlöslichen pharmazeutischen Bindemittels, 2-20 % einer hydrophoben Komponente und 30-90 % Nicotinsäure umfasst.

2. Einsatz nach Anspruch 1, wobei das Medikament mit verzögerter Freisetzung 250 bis 3000 mg Nicotinsäure oder einer aus der Gruppe von d-Glucitolhexanicotinat, Aluminiumnicotinat, Niceritrol, d,1-Alphatocopherylnicotinat und Nicotinylalkoholtartrat gewählten, durch den Körper zu Nicotinsäure verstoffwechselten Verbindung umfasst.

3. Einsatz nach einem der vorstehenden Ansprüche, wobei das Medikament mit verzögerter Freisetzung 5-50 % Gewichtsteile Hydroxypropylmethylcellulose umfasst.

4. Einsatz nach einem der vorstehenden Ansprüche, wobei das Medikament mit verzögerter Freisetzung 1-4 Gew. % eines pharmazeutisch annehmbaren Bindemittels umfasst.

5. Einsatz nach Anspruch 4, wobei das Bindemittel ein Polymer ist, das die wiederholte Einheit 1-Ethenyl-2-pyrrolidon aufweist.

6. Einsatz nach einem der vorstehenden Ansprüche, wobei das Medikament mit verzögerter Freisetzung 0,5-2,0 Gewichtsteile eines Gleitmittels umfasst.

7. Einsatz nach Anspruch 6, dadurch characterisiert, dass das Gleitmittel Stearinsäure oder Magnesiumstearat ist.

8. Medikament mit verzögerter Freisetzung, umfassend Nicotinsäure oder einer aus der Gruppe von d-Glucitolhexanicotinat, Aluminiumnicotinat, Niceritrol, d,1-Alphatocopherylnicotinat und Nicotinylalkoholtartrat gewählten, durch den Körper zu Nicotinsäure verstoffwechselten Verbindung zur Herstellung eines Medikaments mit verzögerter Freisetzung zum Einsatz bei der Behandlung im Wege der oralen Verabreichung einmal pro Tag vor dem Schlafen von Hyperlipidämie, dadurch charakterisiert, dass das Medikament keine Beimischung von 5 - 30 % Hydroxypropylmethylcellulose, 2-15 % eines wasserlöslichen pharmazeutischen Bindemittels, 2-20 % einer hydrophoben Komponente und 30-90 % Nicotinsäure umfasst.

9. Medikament mit verzögerter Freisetzung nach Anspruch 8, wobei das Medikament mit verzögerter Freisetzung 250 bis 3000 mg Nicotinsäure oder einer aus der Gruppe von d-Glucitolhexanicotinat, Aluminiumnicotinat, Niceritrol, d,1-Alphatocopherylnicotinat und Nicotinylalkoholtartrat gewählten, durch den Körper zu Nicotinsäure verstoffwechselten Verbindung umfasst.

10. Medikament mit verzögerter Freisetzung nach Anspruc 8 oder 9, wobei das Medikament mit verzögerter Freisetzung 5-50 % Gewichtsteile Hydroxypropylmethylcellulose umfasst.

11. Medikament mit verzögerter Freisetzung nach einem der Ansprüche 8 bis 10, wobei das Medikament mit verzögerter Freisetzung 1-4 Gew. % eines pharmazeutisch annehmbaren Bindemittels umfasst.

12. Medikament mit verzögerter Freisetzung nach Anspruch 11, wobei das Bindemittel ein Polymer ist, das die wiederholte Einheit 1-Ethenyl-2-pyrrolidon aufweist.

13. Medikament mit verzögerter Freisetzung nach einem der Ansprüche 8 bis 12, wobei das Medikament mit verzögerter Freisetzung 0,5-2,0 Gewichtsteile eines Gleitmittels umfasst.

14. Medikament mit verzögerter Freisetzung nach Anspruch 13, dadurch characterisiert, dass das Gleitmittel Stearinsäure oder Magnesiumstearat ist.

## Revendications

1. Utilisation d'acide nicotinique ou d'un composé métabolisé en acide nicotinique par le corps choisi dans le groupe constitué d'hexanicotinate de d-glucitol, de nicotinate d'aluminium, de nicéritrol, de nicotinate de d,l-alpha-tocophéryle et de tartrate d'alcool de nicotinyle, pour la fabrication d'un médicament à libération prolongée pour une utilisation dans le traitement, par une administration orale une fois par jour avant de dormir, d'une hyperlipidémie **caractérisée en ce que** le médicament ne comprend pas en mélange 5-30% de méthylcellulose d'hydroxypropyle, 2-15% d'un liant pharmaceutique soluble dans l'eau, 2-20% d'un composant hydrophobe et 30-90% d'acide nicotinique.

2. Utilisation selon la revendication 1, dans laquelle ledit médicament à libération prolongée comprend de 250 milligrammes à 3000 milligrammes d'acide nicotinique ou d'un composé métabolisé en acide nicotinique par le corps choisi dans le groupe constitué d'hexanicotinate de d-glucitol, de nicotinate d'aluminium, de nicéritrol, de nicotinate de d,1-alpha-tocophéryle et de tartrate d'alcool de nicotinyle.

3. Utilisation selon l'une quelconque des revendications précédentes, dans laquelle ledit médicament à libération prolongée comprend de 5% à 50% de parties en poids de méthylcellulose d'hydroxypropyle.

4. Utilisation selon l'une quelconque des revendications précédentes, dans laquelle ledit médicament à libération prolongée comprend de 1% à 4% en poids d'un liant pharmaceutiquement acceptable.

5. Utilisation selon la revendication 4, dans laquelle ledit liant est un polymère possédant l'unité de répétition 1-éthényl-2-pyrrolidone.

6. Utilisation selon l'une quelconque des revendications précédentes, dans laquelle ledit médicament à libération prolongée comprend de 0,5 à 2,0 parties en poids d'un lubrifiant.

7. Utilisation selon la revendication 6, **caractérisée en ce que** ledit lubrifiant est l'acide stéarique ou le stéarate de magnésium.

8. Médicament à libération prolongée comprenant de l'acide nicotinique ou un composé métabolisé en acide nicotinique par le corps choisi dans le groupe constitué d'hexanicotinate de d-glucitol, de nicotinate d'aluminium, de nicéritrol, de nicotinate de d,1-alpha-tocophéryle et de tartrate d'alcool de nicotinyle, pour une utilisation dans le traitement, par une administration orale une fois par jour avant de dormir, d'une hyperlipidémie **caractérisée en ce que** le médicament ne comprend pas en mélange 5-30% de méthylcellulose d'hydroxypropyle, 2-15% d'un liant pharmaceutique soluble dans l'eau, 2-20% d'un composant hydrophobe et 30-90% d'acide nicotinique.

9. Médicament à libération prolongée selon la revendication 8, où ledit médicament à libération prolongée comprend de 250 milligrammes à 3000 milligrammes d'acide nicotinique ou d'un composé métabolisé en acide nicotinique par le corps choisi dans le groupe constitué d'hexanicotinate de d-glucitol, de nicotinate d'aluminium, de nicéritrol, de nicotinate de d,1-alpha-tocophéryle et de tartrate d'alcool de nicotinyle.

10. Médicament à libération prolongée selon la revendication 8 ou la revendication 9, où ledit médicament à libération prolongée comprend de 5% à 50% de parties en poids de méthylcellulose d'hydroxypropyle.

11. Médicament à libération prolongée selon l'une quelconque des revendications 8 à 10, où ledit médicament à libération prolongée comprend de 1 % à 4% en poids d'un liant pharmaceutiquement acceptable.

12. Médicament à libération prolongée selon la revendication 11, dans lequel ledit liant est un polymère possédant l'unité de répétition 1-éthényl-2-pyrrolidone.

13. Médicament à libération prolongée selon l'une quelconque des revendications 8 à 12, où ledit médicament à libération prolongée comprend de 0,5 à 2,0 parties en poids d'un lubrifiant.

14. Médicament à libération prolongée selon la revendication 13, **caractérisé en ce que** ledit lubrifiant est l'acide stéarique ou le stéarate de magnésium.
